(19) Europäisches Patentamt
European Patent Office
Office européen des brevets

(11) **EP 1 908 401 A1**

(12) **DEMANDE DE BREVET EUROPEEN**

(43) Date de publication:
**09.04.2008 Bulletin 2008/15**

(51) Int Cl.:
***A61B 5/024*** (2006.01)

(21) Numéro de dépôt: **06121865.7**

(22) Date de dépôt: **06.10.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(71) Demandeur: **ETA SA Manufacture Horlogère Suisse**
**2540 Grenchen (CH)**

(72) Inventeurs:
• **Renevey, Philippe**
**1005, Lausanne (CH)**
• **Vetter, Rolf**
**1116, Cottens (CH)**
• **Galli, Reto**
**3012, Bern (CH)**

(74) Mandataire: **Robert, Vincent et al**
**ICB S.A.**
**Rue des Sors 7**
**2074 Marin (CH)**

(54) **Méthode et dispositif de mesure d'une pulsation cardiaque lors de la pratique d'un sport rythmique**

(57) L'invention concerne une méthode de mesure d'une pulsation cardiaque durant une activité sportive de type rythmique, consistant principalement à :
- transmettre un rayonnement lumineux dans des tissus organiques,
- détecter un signal optique issu de l'interaction du rayonnement lumineux avec les tissus organiques, le signal optique comprenant une composante relative à la pulsation cardiaque et une composante relative au mouvement,
- détecter un signal d'accélération contenant une indication relative au mouvement effectué durant l'activité sportive,
- effectuer un rehaussement du signal optique de manière à en éliminer la composante relative au mouvement effectué, et
- calculer la pulsation cardiaque à partir du signal optique rehaussé.

Selon l'invention, le rehaussement du signal optique comprend principalement les étapes suivantes :
- déterminer une fréquence fondamentale du mouvement à partir du signal d'accélération,
- déterminer des fréquences harmoniques du mouvement, et
- éliminer du signal optique les fréquences du mouvement déterminées.

Fig. 3

**Description**

La présente invention se rapporte au domaine du traitement du signal. Elle concerne plus particulièrement une méthode et un dispositif intégré de mesure d'une pulsation cardiaque durant la pratique d'une activité sportive de type rythmique.

Les dispositifs de mesure du rythme cardiaque comportent généralement une sonde externe, fixée, par exemple, à la poitrine ou à l'oreille, et connectée à une unité de traitement du signal et d'affichage. Ces dispositifs sont relativement encombrants et incommodes.

Récemment, des dispositifs intégrés ont fait leur apparition. Par dispositif intégré, on entend un dispositif formé d'un unique module effectuant, au même point, la mesure d'un signal, son traitement et l'affichage du résultat. De tels dispositifs sont, par exemple, portés au poignet à la façon d'une montre. Leur fonctionnement est basé sur une technique optique de mesure d'une donnée physiologique appelée photoplethysmographie (ou PPG). Le principe en est le suivant : Une source optique émet un rayonnement infrarouge se propageant à l'intérieur des tissus humains dans lesquels il subit de la diffusion et de l'absorption. L'interaction entre le rayonnement et les tissus dépend du type et du volume de tissu traversé. Un détecteur optique mesure le signal en sortie des tissus. Ce signal contient une information sur les tissus traversés. En l'absence de mouvement du porteur, il comporte une composante continue, issue des tissus statiques, et une composante périodique, issue des tissus pulsatiles, typiquement le sang. Cette dernière composante permet la mesure du rythme cardiaque.

Un inconvénient lié à la méthode de mesure du rythme cardiaque par PPG est sa sensibilité aux mouvements du sujet sur lequel la mesure est pratiquée. Ces mouvements génèrent des artefacts dont la contribution au signal excède souvent la contribution utile de la pulsation cardiaque, d'un ordre de grandeur. Le signal optique doit ainsi être traité de manière à éliminer la contribution due aux mouvements. On parle de rehaussement du signal optique. A cet effet, les dispositifs intègrent généralement un accéléromètre à trois dimensions apte à délivrer un signal représentatif du mouvement effectué au point où le signal optique est mesuré.

Différentes méthodes de rehaussement du signal optique à l'aide du signal d'accélération ont été divulguées à ce jour. On citera, par exemple, le document US 7 018 338 qui décrit une méthode basée sur la modélisation non linéaire du signal d'accélération. Une autre méthode, décrite dans le document WO 2006/044677, transpose les signaux optique et d'accélération, du domaine temporel au domaine fréquentiel par transformation de Fourier (FFT), en vue d'éliminer les artefacts liés au mouvement.

De telles méthodes de rehaussement du signal optique sont extrêmement lourdes et nécessitent une puissance de calcul considérable. Pour des questions de coût et d'encombrement, des solutions plus simples sont à privilégier. La présente invention se base sur une analyse rigoureuse du mouvement sportif pour proposer une méthode et un dispositif de détection du rythme cardiaque simplifiés, robustes et performants.

Plus précisément, l'invention concerne une méthode de mesure d'une pulsation cardiaque durant une activité sportive de type rythmique impliquant un mouvement périodique, consistant principalement à :

-   transmettre un rayonnement lumineux dans des tissus organiques,
-   détecter un signal optique issu de l'interaction dudit rayonnement lumineux avec les tissus organiques, le signal optique comprenant une composante relative à la pulsation cardiaque et une composante relative au mouvement effectué durant l'activité sportive,
-   détecter un signal d'accélération contenant une indication relative au mouvement effectué durant l'activité sportive,
-   effectuer un rehaussement du signal optique de manière à en éliminer la composante relative au mouvement effectué, et
-   calculer la pulsation cardiaque à partir du signal optique ainsi rehaussé,

Selon l'invention, le rehaussement du signal optique comprend principalement les étapes suivantes :

-   déterminer la fréquence fondamentale du mouvement,
-   déterminer les harmoniques correspondant à la fréquence fondamentale, et
-   éliminer les fréquences ainsi déterminées du signal optique.

Grâce à ces caractéristiques, la méthode de mesure du rythme cardiaque selon l'invention peut gagner en rapidité, en simplicité et en robustesse.

L'invention concerne également un dispositif intégré de mesure d'une pulsation cardiaque durant une activité sportive de type rythmique, pour la mise en oeuvre d'une telle méthode, comportant :

-   une source lumineuse,
-   un photo-détecteur fournissant un signal optique comprenant une composante relative à la pulsation cardiaque et une composante relative au mouvement effectué durant l'activité sportive,

- un accéléromètre fournissant un signal d'accélération contenant une indication relative au mouvement effectué durant l'activité sportive, et
- un processeur apte à effectuer un rehaussement du signal optique de manière à en éliminer la composante relative au mouvement effectué, et calculer la pulsation cardiaque à partir du signal optique rehaussé,

ledit dispositif intégré étant caractérisé en ce que le rehaussement du signal optique comprend principalement les étapes suivantes :

- déterminer une fréquence fondamentale du mouvement à partir du signal d'accélération,
- déterminer des fréquences harmoniques du mouvement, et
- éliminer du signal optique les fréquences du mouvement déterminées.

D'autres caractéristiques et avantages de la présente invention ressortiront plus clairement de la description détaillée qui suit d'un exemple de réalisation d'un dispositif de mesure d'un rythme cardiaque selon l'invention, cet exemple étant donné à titre purement illustratif et non limitatif seulement, en liaison avec le dessin annexé sur lequel :

- les figures 1 et 2 sont des vues respectivement de dessous et en coupe du dispositif selon l'invention,
- la figure 3 est un diagramme illustrant un algorithme de calcul du rythme cardiaque utilisé par le dispositif selon l'invention, et
- la figure 4 se rapporte à une étape de l'algorithme représenté en figure 3.

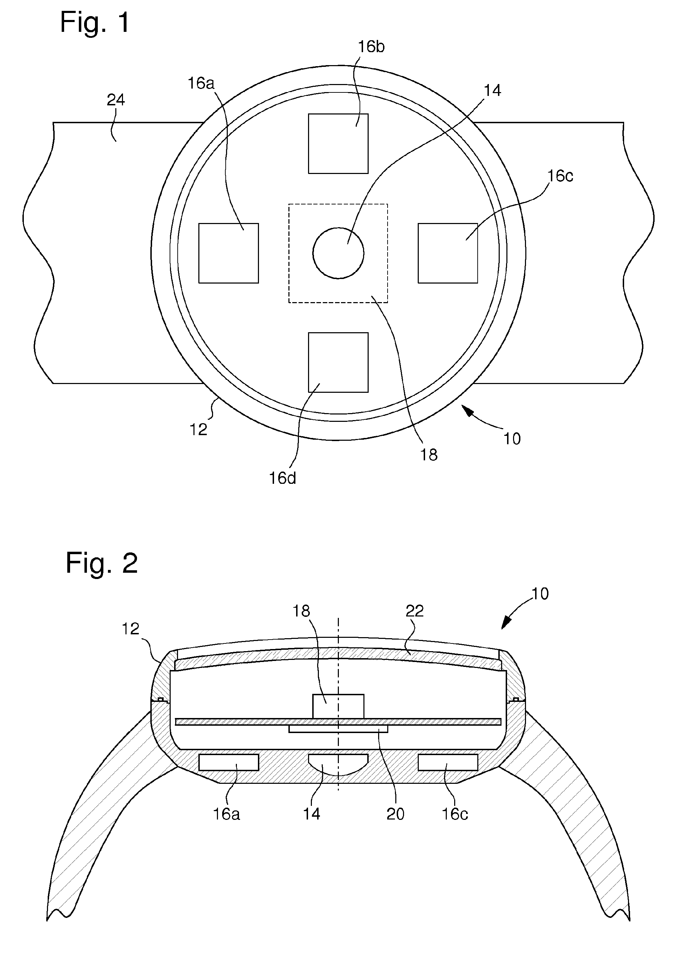

Le dispositif intégré de mesure d'un rythme cardiaque représenté schématiquement sur les figures 1 et 2 et désigné par la référence générale 10, comporte classiquement un boîtier 12 comprenant une source lumineuse 14 destinée à transmettre un rayonnement infrarouge à des tissus humains, et quatre photo-détecteurs 16a, 16b, 16c et 16d, tels que des photodiodes, disposés autour de la source lumineuse 14 de manière à recevoir le rayonnement infrarouge après son interaction avec les tissus. Le dispositif 10 comporte également un système de détection du mouvement 18, tel qu'un accéléromètre à trois dimensions, et un processeur 20 agencé pour recevoir et traiter les signaux issus des photo-détecteurs 16a, 16b, 16c, 16d, et de l'accéléromètre 18. Un module d'affichage 22, tel qu'un écran LCD, intégré au boîtier 12, est destiné à l'affichage des paramètres physiologiques mesurés, en particulier du rythme cardiaque. Le boîtier 12 est, en outre, muni d'un bracelet 24 pour la fixation du dispositif 10 au poignet à la façon d'une montre.

Le dispositif 10 est destiné à la mesure d'un rythme cardiaque d'un sportif pratiquant un sport de type rythmique tel que la marche, la course à pied, le cyclisme, la natation etc, ou tout sport associé à un mouvement périodique. Son fonctionnement est le suivant : La source lumineuse 14 émet un rayonnement infrarouge qui interagit avec les tissus humains. Les photo-détecteurs 16a, 16b, 16c et 16d captent un signal optique contenant une information sur les tissus traversés. Parallèlement, l'accéléromètre 18 capte un signal d'accélération contenant une information sur le mouvement sportif effectué. Les signaux optique et d'accélération sont traités par le processeur 20, en vue d'extraire une information utile sur un paramètre physiologique, en particulier un rythme cardiaque. A cet effet, le processeur 20 met en oeuvre un algorithme de traitement du signal original et performant, fondé sur une observation et une analyse minutieuses du mouvement sportif périodique.

Un tel mouvement est globalement complexe. Il est formé d'une combinaison de sous-mouvements : les sous-mouvements des jambes, des bras, du torse etc...Il a été observé, dans le cadre de la présente invention, que chacun de ces sous-mouvements est de type harmonique, c'est-à-dire caractérisé par une fréquence fondamentale $f_0$, et des fréquences harmoniques $f_1$, $f_2$, ... $f_i$, multiples de la fréquence fondamentale $f_0$. De plus, les sous-mouvements ne sont pas indépendants les uns des autres : Leurs fréquences fondamentales sont elles même multiples les unes des autres, de sorte que le mouvement global est un mouvement harmonique de fréquence fondamentale la plus petite des fréquences fondamentales des différents sous-mouvements. Une telle observation simplifie considérablement le traitement du signal d'accélération. En effet, il s'avère suffisant, sur la base de cette observation, de déterminer la fréquence fondamentale $f_0$ du mouvement sportif, pour obtenir l'ensemble des fréquences du mouvement par simple multiplication. Le calcul d'un spectre en fréquence complet du signal d'accélération, est ainsi inutilement lourd et complexe, et sa forte consommation en puissance de calcul est injustifiée.

On notera toutefois que seul un mouvement global idéal est rigoureusement harmonique. Dans le cas réel, le mouvement global est sensiblement harmonique car les fréquences fondamentales des différents sous-mouvements ne sont pas rigoureusement multiples les unes des autres, et de petites variations existent. On verra dans la suite de cet exposé, comment de telles variations sont prises en compte dans l'estimation de la pulsation cardiaque.

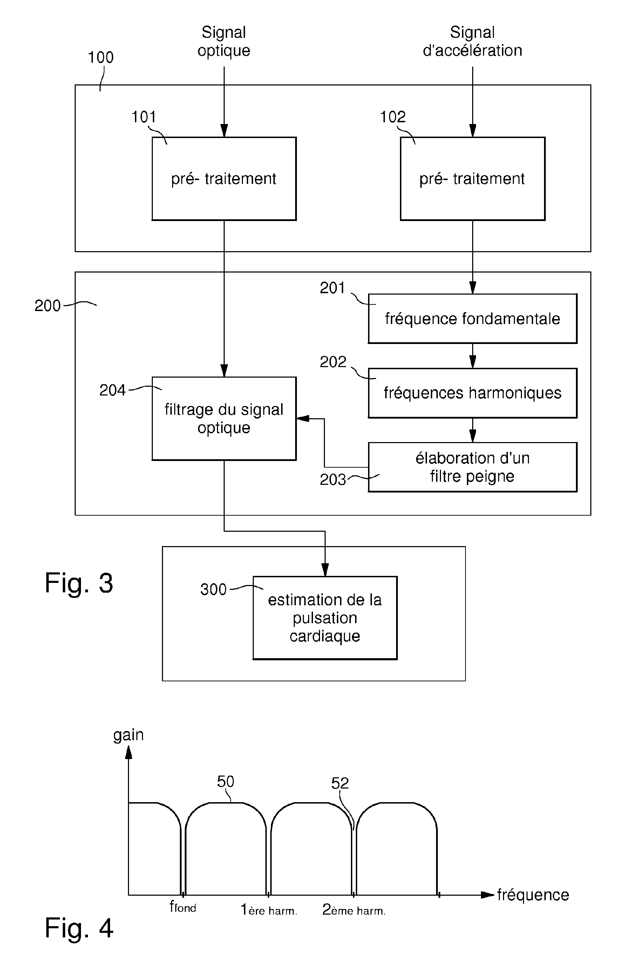

L'algorithme selon l'invention, illustré schématiquement en figure 3, est basé sur l'analyse précédente. Il comprend trois blocs principaux respectivement de prétraitement 100, de rehaussement du signal optique 200, et d'estimation de la pulsation cardiaque 300, comportant chacun un certain nombre de sous-blocs, et détaillés ci-dessous.

### a. Prétraitement 100

Les signaux optique et d'accélération issus respectivement des photo-détecteurs 16a, 16b, 16c, 16d, et de l'accéléromètre 18, subissent en premier lieu un prétraitement, symbolisé respectivement par deux sous-blocs 101 et 102 appartenant au module 100. Le prétraitement 101 consiste généralement, pour le signal optique, en une première étape d'élimination de la composante continue du signal, suivie d'une deuxième étape de filtrage en vue de réduire le domaine de fréquences aux valeurs compatibles avec un rythme cardiaque. Concernant le signal d'accélération, on procède essentiellement lors de l'étape de prétraitement 102 à la suppression de la composante continue. On notera que le prétraitement des signaux optique et d'accélération peut être effectué en mode analogique ou numérique.

### b. Rehaussement du signal optique 200

Le module de rehaussement du signal optique 200 comporte un premier sous-bloc 201 de détermination de la fréquence fondamentale du mouvement à partir du signal d'accélération prétraité, selon l'idée de base de la présente invention que l'extraction de ce seul paramètre est nécessaire.

On utilisera avantageusement pour l'estimation de la fréquence fondamentale du mouvement, la fonction d'auto-corrélation, bien connue de l'homme de métier. D'après le théorème de Wiener-Khintchine, le spectre de la fonction d'auto-corrélation d'un signal est équivalent au spectre de puissance du signal original. Autrement dit, la fonction d'auto-corrélation d'un signal contient la même information fréquentielle que le signal d'origine sans l'information de la phase. En conséquence, pour un signal harmonique, dont les fréquences $f_i$ sont des multiples de la fréquence fondamentale $f_0$, la fonction d'auto-corrélation $r_{\gamma\gamma}$ du signal peut s'écrire sous la forme suivante :

$$r_{\gamma\gamma}(l) = \sum_{n=1}^{M} A_n \cdot \cos(2\pi n f_0 l) \qquad (1)$$

Il ressort de l'équation (1) que la fonction d'auto-corrélation d'un signal harmonique, et en particulier la fonction d'auto-corrélation $r_{\gamma\gamma}$ du signal d'accélération, présente un maximum en $l = 1/f_0$, où $f_0$ est la fréquence fondamentale du signal.

Le sous-bloc 201 de détermination de la fréquence fondamentale procède donc, dans une première étape, au calcul de la fonction d'auto-corrélation du signal d'accélération, puis, dans une seconde étape, à la détection du maximum de la fonction d'auto-corrélation. Dans certains cas, afin d'augmenter la résolution de la détection du maximum et la précision du résultat, on peut effectuer, à l'intérieur du sous-bloc 201, une approximation polynômiale de la fonction d'auto-corrélation autour du maximum.

Bien entendu, toute autre méthode mathématique permettant de déterminer la fréquence fondamentale $f_0$ du mouvement est utilisable. On citera, par exemple, les méthodes de transformation telles que la transformation de Fourier (FFT), la transformation de cosinus (DCT), ou les méthodes paramétriques. Toutefois, ces méthodes ne se limitent pas au calcul du paramètre utile, la fréquence fondamentale $f_0$, mais, au contraire, portent sur un grand nombre de paramètres. Elles sont par nature lourdes, et nécessitent une puissance de calcul considérable. C'est pourquoi, le calcul de la fréquence fondamentale $f_0$ par la fonction d'auto-corrélation est à privilégier.

A la suite du sous-bloc 201 de détermination de la fréquence fondamentale $f_0$, un deuxième sous-bloc 202 concerne le calcul des harmoniques $f_1$, $f_2$,... $f_i$. Les harmoniques sont obtenues par simple multiplication de la fréquence fondamentale $f_0$ par des entiers naturels (1, 2, 3, ...).

Un troisième sous-bloc 203 fait suite au sous-bloc 202 de calcul des harmoniques. Ce sous-bloc 203 se rapporte à l'élaboration d'un filtre en peigne tel qu'illustré en figure 4. Un tel filtre comporte classiquement de larges bandes passantes 50 et d'étroites bandes de réjection 52 centrées sur la fréquence fondamentale $f_0$ du mouvement et ses harmoniques $f_1$, $f_2$,...$f_i$. La largeur des bandes de réjection est non nulle et fixée par un jeu de paramètres ($\beta_0$, $\beta_1$, $\beta_2$, ... $\beta_i$). Les paramètres ($\beta_0$, $\beta_1$, $\beta_2$, ...$\beta_i$) du filtre en peigne permettent de prendre en compte les petites variations de la fréquence fondamentale et des fréquences harmoniques dues à l'imperfection du mouvement global réel. Avantageusement, le jeu de paramètres $\beta_i$ est déterminé pour une activité donnée. Pour plus de précisions sur les paramètres $\beta_i$, on se reportera à la demande conjointe intitulée 'Méthode et dispositif intégré de mesure d'une pulsation cardiaque' déposée en date du 06 Octobre 2006 au nom de ETA S.A. Manufacture Horlogère Suisse.

Enfin, un sous-bloc 204 de filtrage du signal optique, est relié d'une part au bloc 203 d'élaboration du filtre en peigne, et d'autre part, au sous-bloc 101 de prétraitement du signal optique. Il correspond à l'opération de filtrage du signal optique à l'aide du filtre en peigne élaboré à partir des fréquences $f_i$ du mouvement. Après l'opération de filtrage réalisée en 204, les artefacts liés au mouvement sont éliminés du signal optique.

**-c. Estimation de la pulsation cardiaque 300**

Le signal optique rehaussé est filtré, à l'aide d'un filtre passe-bande adaptatif, ou d'un algorithme de réduction du bruit de type PCA tel que décrit dans le brevet US 7 018 338. Puis, la pulsation cardiaque est estimée à partir du signal optique rehaussé filtré. Pour cela on utilise, par exemple, la méthode du 'zero-crossing' en terminologie anglaise, c'est-à-dire la méthode du passage à zéro, bien connue de l'homme de métier.

On a ainsi décrit un dispositif et une méthode de mesure d'une pulsation cardiaque dont les performances sont augmentées grâce à une méthode de rehaussement du signal optique basée sur une analyse poussée du mouvement sportif périodique.

Bien entendu, la méthode et le dispositif selon l'invention ne se limitent pas au mode de réalisation qui vient d'être décrit et diverses modifications et variantes simples peuvent être envisagées par l'homme du métier sans sortir du cadre de l'invention tel que défini par les revendications annexées.

**Revendications**

**1.** Méthode de mesure d'une pulsation cardiaque durant une activité sportive de type rythmique impliquant un mouvement périodique, consistant principalement à :

- transmettre un rayonnement lumineux dans des tissus organiques,
- détecter un signal optique issu de l'interaction dudit rayonnement lumineux avec les tissus organiques, ledit signal optique comprenant une composante relative à la pulsation cardiaque et une composante relative audit mouvement effectué durant ladite activité sportive,
- détecter un signal d'accélération contenant une indication relative audit mouvement effectué durant ladite activité sportive,
- effectuer un rehaussement du signal optique de manière à en éliminer la composante relative au mouvement effectué, et
- calculer ladite pulsation cardiaque à partir du signal optique rehaussé, ladite méthode étant **caractérisée en ce que** ledit rehaussement du signal optique comprend principalement les étapes suivantes :

- déterminer une fréquence fondamentale du mouvement à partir dudit signal d'accélération,
- déterminer des fréquences harmoniques du mouvement, et
- éliminer dudit signal optique les fréquences du mouvement déterminées.

**2.** Méthode selon la revendication 1, **caractérisée en ce que** seule ladite fréquence fondamentale du mouvement est déterminée à partir dudit signal d'accélération, et **en ce que** les fréquences harmoniques sont déterminées à partir de ladite fréquence fondamentale.

**3.** Méthode selon l'une des revendications 1 et 2, **caractérisée en ce que** ladite détermination de la fréquence fondamentale du mouvement est basée sur le calcul d'une fonction d'auto-corrélation du signal d'accélération, suivi d'une détection des maxima.

**4.** Méthode selon l'une des revendications 1 à 3, **caractérisée en ce que** les fréquences harmoniques sont déterminées par multiplication de la fréquence fondamentale par des entiers naturels (1, 2, 3, ...).

**5.** Méthode selon l'une des revendications 1 à 4, **caractérisée en ce qu'**un filtre peigne comprenant des bandes passantes et des bandes de réjection est construit à partir des fréquences du mouvement déterminées, lesdites bandes de réjection étant centrées sur lesdites fréquences du mouvement, et **en ce que** ledit filtre peigne est appliqué audit signal optique de manière à le rehausser.

**6.** Dispositif intégré de mesure d'une pulsation cardiaque durant une activité sportive de type rythmique, pour la mise en oeuvre de la méthode selon les revendications 1 à 5, comportant :

- une source lumineuse,
- un photo-détecteur fournissant un signal optique comprenant une composante relative à ladite pulsation cardiaque et une composante relative au mouvement effectué durant ladite activité sportive,

- un accéléromètre fournissant un signal d'accélération contenant une indication relative au mouvement effectué durant ladite activité sportive, et
- un processeur apte effectuer un rehaussement dudit signal optique de manière à en éliminer la composante relative au mouvement effectué, et calculer ladite pulsation cardiaque à partir du signal optique rehaussé,

**caractérisé en ce que** ledit rehaussement du signal optique comprend principalement les étapes suivantes :

- déterminer une fréquence fondamentale du mouvement à partir dudit signal d'accélération,
- déterminer des fréquences harmoniques du mouvement, et
- éliminer dudit signal optique les fréquences du mouvement ainsi déterminées.

## Fig. 1

## Fig. 2

Signal
optique

Signal
d'accélération

100

101 pré- traitement

102 pré- traitement

200

201 fréquence fondamentale

202 fréquences harmoniques

204 filtrage du signal optique

203 élaboration d'un filtre peigne

**Fig. 3**

300 estimation de la pulsation cardiaque

gain

50

52

$f_{fond}$    1ère harm.    2ème harm.    fréquence

**Fig. 4**

**Office européen
des brevets**

**RAPPORT DE RECHERCHE EUROPEENNE**

Numéro de la demande

EP 06 12 1865

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 0 659 384 A1 (SEIKO INSTR INC [JP]) 28 juin 1995 (1995-06-28) * le document en entier * ----- | 1-6 | INV. A61B5/024 |
| X | EP 0 922 433 A1 (SEIKO EPSON CORP [JP]; SEIKO INSTR INC [JP]) 16 juin 1999 (1999-06-16) * le document en entier * ----- | 1-6 | |
| X | US 2004/236233 A1 (KOSUDA TSUKASA [JP] ET AL) 25 novembre 2004 (2004-11-25) | 1,6 | |
| A | * le document en entier * ----- | 2-5 | |
| A | EP 0 941 694 A1 (SEIKO EPSON CORP [JP]) 15 septembre 1999 (1999-09-15) * alinéas [0002], [0007], [0008] * * alinéas [0036] - [0045]; figures 1-4 * * alinéas [0063] - [0067]; figure 16 * * alinéas [0098] - [0101] * * alinéas [0107] - [0118] * * alinéas [0159] - [0167] * ----- | 1-6 | |

**DOMAINES TECHNIQUES RECHERCHES (IPC)**

A61B

Le présent rapport a été établi pour toutes les revendications

| Lieu de la recherche | Date d'achèvement de la recherche | Examinateur |
|---|---|---|
| La Haye | 28 mars 2007 | Dhervé, Gwenaëlle |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

&: membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 06 12 1865

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-03-2007

| Document brevet cité au rapport de recherche | | Date de publication | Membre(s) de la famille de brevet(s) | | Date de publication |
|---|---|---|---|---|---|
| EP 0659384 | A1 | 28-06-1995 | DE | 69429905 D1 | 28-03-2002 |
| | | | DE | 69429905 T2 | 14-08-2002 |
| | | | JP | 2816944 B2 | 27-10-1998 |
| | | | JP | 7227383 A | 29-08-1995 |
| | | | US | 5697374 A | 16-12-1997 |
| EP 0922433 | A1 | 16-06-1999 | JP | 3584143 B2 | 04-11-2004 |
| | | | JP | 10258038 A | 29-09-1998 |
| | | | WO | 9841143 A1 | 24-09-1998 |
| | | | US | 6155983 A | 05-12-2000 |
| US 2004236233 | A1 | 25-11-2004 | CN | 1531901 A | 29-09-2004 |
| | | | JP | 3815448 B2 | 30-08-2006 |
| | | | JP | 2004283228 A | 14-10-2004 |
| EP 0941694 | A1 | 15-09-1999 | WO | 9912469 A1 | 18-03-1999 |
| | | | JP | 3689914 B2 | 31-08-2005 |
| | | | US | 6198951 B1 | 06-03-2001 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- US 7018338 B

- WO 2006044677 A